# EUROPEAN PATENT APPLICATION

(11) **EP 4 807 753 A1**
(43) Date of publication of application: **16.09.2026**
(21) Application number: 26158827.1
(22) Date of filing: 16.02.2026
(51) Int. Cl.: G16H 10/20, G06N 20/00

(54) **CALIBRATING A CLINICAL TRIAL MACHINE LEARNING-BASED AGENT**

(30) Priority: 14.03.2025 US 202563771935 P
(71) Applicant: Medidata Solutions, Inc., New York, NY 10014 (US)
(72) Inventor: NOUIRA, Marouane, New York, NY, 10014 (US); XU, Sabrina, New York, NY, 10014 (US); TANG, Kei, New York, NY, 10014 (US); CHOKDA, Pranav, New York, NY, 10014 (US)
(74) Representative: Fish & Richardson P.C.

(57) **Abstract**

A method implemented by a data processing system for calibrating an artificial intelligence model to provide near real-time outputs during a clinical trial study, comprising: detecting that the one or more attributes for which the one or more values are modified are of a specified type for which an artificial intelligence (AI) model is calibrated; responsive to the detecting, generating a set of input and output data for calibrating the AI model; inputting the set of input and output data into the AI model; calibrating the AI model for the clinical trial study to provide one or more outputs that account in near real-time for the one or more modifications to the one or more values of the clinical trial study; and causing rendering of a graphical user interface with an input portion for inputting a query related to the clinical trial study.

## Description

### CLAIM OF PRIORITY

This application claims priority under 35 U.S.C. §119(e) to U.S. Patent Application Serial No. 63/771,935, filed on March 14, 2025, the entire contents of which are hereby incorporated by reference.

### BACKGROUND

In general, clinical trials are prospective biomedical or behavioral research studies that are designed to answer specific questions about biomedical or behavioral interventions. For example, clinical trials can be performed to evaluate the safety and/or efficacy of vaccines, drugs, dietary choices, dietary supplements, and/or medical devices.

### SUMMARY

The techniques described herein address the following technical problems in clinical study data management: (1) inefficient information retrieval due to data heterogeneity, volume, and lack of natural language interfaces; (2) difficulty in understanding and interpreting raw clinical data due to complex data structures and lack of context; and (3) the challenge of maintaining up-to-date knowledge of evolving study data due to dynamic updates and the lack of continuous learning in existing systems.

The techniques described herein employ continuous fine-tuning of a machine learning (ML) model, including, e.g., a large language model (LLM). The techniques described herein also employ automated data capture from diverse sources including audit trails, raw data to human-readable translation during fine-tuning, a natural language interface, and real-time information retrieval. This approach solves the problem of inefficient information retrieval by providing a natural language interface and study-specific training. It addresses the difficulty of interpreting raw data by teaching the LLM to translate it into human-readable form. Finally, continuous fine-tuning ensures the system's knowledge is always up-to-date, solving the problem of maintaining current information in dynamic clinical studies.

The techniques described herein offer several advantages over existing solutions. They increase efficiency in information retrieval by allowing natural language queries, reducing the time and effort required compared to a traditional clinical data management system (CDMS) and keyword searches. The techniques described herein improve accessibility of complex data by translating raw data into human-readable explanations, making it accessible to a broader audience than direct access or rule-based systems. The continuous fine-tuning process provides real-time, up-to-date information and insights, a significant advantage over static reports and one-time fine-tuned models. Finally, the automated data capture and training significantly reduce manual effort compared to traditional methods.

In some implementations, a method implemented by a data processing system for calibrating an artificial intelligence model to provide near real-time outputs during a clinical trial study, comprises receiving, at a data processing system, an indication of one or more modifications to one or more values of one or more attributes of a participant in the clinical trial study, with the indication specifying an identifier representing the participant in the clinical trial study; detecting that the one or more attributes for which the one or more values are modified are of a specified type for which an artificial intelligence (AI) model is calibrated; responsive to the detecting, generating, in accordance with the received indication, a set of input and output data for calibrating the AI model, with the output specifying an appropriate response to the input, and with the input being in accordance with the one or more attributes for which the one or more values are modified; inputting the set of input and output data into the AI model; based on the inputting of the set of input and output data, calibrating the AI model for the clinical trial study to provide one or more outputs that account in near real-time for the one or more modifications to the one or more values of the clinical trial study; causing rendering of a graphical user interface with an input portion for inputting a query related to the clinical trial study; receiving, through the graphical user interface, a query for one or more values of the one or more attributes of the participant, with the query specifying the identifier; transmitting the query to the calibrated AI model; receiving, from the calibrated AI model, an output that is in accordance with the one or more modifications to the one or more values, with the output being in near real-time relative to receipt of the indication; and updating the graphical user interface with one or more visualizations that are in accordance with the received output, wherein the one or more visualizations are juxtaposed to the input portion.

In this implementation, the one or more attributes are one or more first attributes, wherein the identifier is a primary key, and wherein generating the set of input data comprises: identifying one or more second attributes to be included in the set of input data; searching data structures stored in memory for one or more foreign keys with a foreign-primary key relationship to the primary key; based on the searching, identifying one or more data structures stored in memory with one or more foreign keys with a foreign-primary key relationship to the primary key; retrieving, from those one or more identified data structures, one or more values of the one or more second attributes, with the one or more retrieved values being associated with the foreign key; generating input data in accordance with one or more modified values of the one or more first attributes and one or more values of the one or more second attributes. The method includes continuously calibrating the AI model during the clinical trial study, based on detection of one or more additional modifications of one or more specified types. The calibrating includes fine-tuning the AI model with the set of input and output data. The method includes storing optimization rules specifying one or more types of modifications or one or more types of attributes that trigger a calibration of the AI model; and executing the optimization rules. The method includes polling a hardware storage device for new audit data, with new audit data being audit data stored in the hardware storage device after a specified time. The calibrating comprises: training one or more specified parameters of the AI model in accordance with the generated set; or performing in-context fine-tuning of the AI model in accordance with the generated set.

Other implementations are directed to systems, devices, and devices for performing some or all of the method. Other implementations are directed to one or more non-transitory computer-readable media including one or more sequences of instructions which when executed by one or more processors causes the performance of some or all of the method.

The details of one or more embodiments are set forth in the accompanying drawings and the description. Other features and advantages will be apparent from the description and drawings, and from the claims.

### BRIEF DESCRIPTION OF THE DRAWINGS

FIG. 1A shows an example system for calibrating a clinical trial ML-based agent.
FIG. 1B shows an example data processing system.
FIGS. 2-5 show example graphical user interfaces.
FIG. 6 illustrates an example process for calibrating a clinical trial ML-based agent.
FIG. 7 depicts an example computing system, according to implementations of the present disclosure.

Like reference numbers and designations in the various drawings indicate like elements.

### DETAILED DESCRIPTION

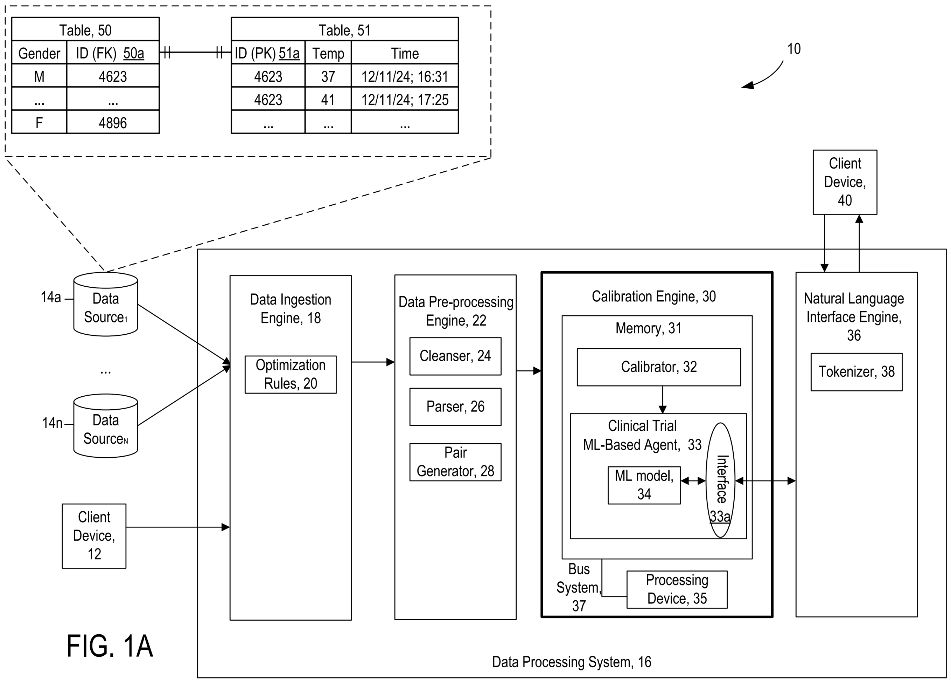

Referring to FIG. 1A, environment 10 for calibrating an artificial intelligence model to provide near real-time outputs during a clinical trial study is shown. In this example, environment 10 includes client devices 12, 40, data sources 14a...14n, and data processing system 16. Data processing system 16 includes data ingestion engine 18, data pre-processing engine 22, calibration engine 30 (with calibrator 32 and clinical trial ML-based agent 33 (with ML model 34), amongst others), and natural language interface engine 36. As used herein, calibrating clinical trial ML-based agent 33 includes (or is) calibrating ML model 34.

In operation, client device 12 transmits ingestion instructions to data ingestion engine 18. Generally, data ingestion includes the process of collecting and moving data from various sources into a central location for storage and analysis. The ingestion instructions may specify a time or times at which data ingestion engine 18 ingests or accesses data from data sources 14a...14n. In this example, data ingestion engine 18 automatically collects data from data sources 14a...14n. In this example, data source 14a stores tables 50, 51, each of which includes cells (e.g., fields) specifying values, e.g., of data collected during a clinical trial. Table 50 specifies a gender of the participants in the clinical trial. In table 50, each participant is identified by an identifier, as shown in cell 50a. Table 51 specifies the temperature of the various participants in the clinical trial and the time at which data specifying the temperature was collected. Each participant represented in table 51 is also identified by an identifier, as shown in cell 51a. In this example, foreign key (FK) - primary key (PK) relationships are used to traverse cells in tables 50, 51 to identify cells pertaining to a particular participant, related cells, and so forth. In this example, cell 51a represents a PK and cell 50a represents a FK. As such, data processing system 16 can identify in table 51 a temperature for a particular participant. Data processing system 16 looks-up in table 51 a value of the PK for that particular participant and can then query table 50 for data associated with a related FK (e.g., a FK having a value of the PK).

There are various types of data sources, including, e.g., a study protocol source that includes information related to how a study will be conducted, a study metadata source that includes metadata associated with how the data will be captured for the study, a clinical data (e.g., case report form (CRF) data) source that stores clinical data (that is accessible to data ingestion engine 18 using database connectors to access and retrieve the data), and an audit trail logs source (e.g., a source with logs that are in accordance with Operational Data Model (ODM) Extensible Markup Language (XML)). The audit trail logs source is a dynamic data source. Data ingestion engine 18 parses XML files and extracts relevant information about data changes, timestamps, and user actions. There are also other data sources, including, e.g., sensors and wearables for recording measurements (e.g., temperature).

In some examples, data ingestion engine 18 polls data sources 14a...14n from time to time for changes to data stored in data sources 14a...14n. However, in some examples, not every change triggers calibration (e.g., fine-tuning) of ML model 34. Data ingestion engine 18 stores optimization rules 20 that specify the types of changes that trigger a fine-tuning of ML model 34. In some examples, optimization rules 20 specify a rule-based model, in which certain types of data have a higher weight as compared to others. In this example, data has to be associated with at least a threshold weight to trigger fine-tuning of ML model 34. Data associated with a weight beneath the threshold does not trigger fine-tuning of the ML model 34.

In another example, optimization rules 20 use a trained ML model to determine which data (or which types of data) trigger a fine-tuning of ML model 34. In this example, data processing system 16 may train another ML model to find important datum amongst a bunch of changes. In still another example, data ingestion engine 18 may specify that ML model 34 is fine-tuned for all updates to data.

In an example, data ingestion engine 18 detects a change in data in at least one of data sources 14a...14n. In response, data ingestion engine 18 applies optimization rules 20 to the changed data to determine whether the change is of a type for which ML model 34 is fine-tuned. In this case, data ingestion engine 18 determines that ML model 34 is fine-tuned for the change. In response, data ingestion engine 18 transmits the changed data and a notification of the change to data pre-processing engine 22.

Data pre-processing engine 22 prepares the data for fine-tuning. In particular, data pre-processing engine 22 incudes cleanser 24 that cleanses the data (i.e., the data with the change), e.g., by removing irrelevant characters, handling missing values, standardizing data formats, and so forth. In some examples, data is structured with fields. Cleanser 24 is configured to detect or otherwise identify a field, identify a type of data in those fields and - based on the type of data - detect whether the data in the field has irrelevant characters, missing values, and so forth.

Data pre-processing engine 22 also includes parser 26 to parse the data and generate - based on the parsed data - a human-readable version of the data. Cleanser 24 passes the cleansed data to parser 26. In this example, parser 26 acts as a ground truth generator. For example, for each raw data entry (e.g., an ODM XML audit entry from one of data sources 14a...14n), parser 26 generates a natural language version (e.g., the ground truth). For example:
▪ **Raw Data (ODM XML):**
   <ItemData><ItemOID>SystolicBP</ItemOID><Value>120</Value><Tim estamp>2024-01-15T10:00:00</Timestamp><ActionType>Insert</ActionType></ItemData >
**▪ Ground Truth:** "SystolicBP was inserted with a value of 120 on 2024-01-15 at 10:00:00."

Parser 26 transmits the natural language version to pair generator 28 for data formatting. In particular, pair generator 28 formats the data into a suitable format for fine-tuning ML model 34 (e.g., question-answer pairs, prompt-completion pairs, and so forth). Pair generator 28 transmits the pairs to calibration engine 30, which performs fine-tuning (e.g., continuously, intermittently, and so forth) of ML model 34. In this example, ML model 34 includes a study specific ML model, e.g., a domain specific LLM where the domain is the particular clinical trial study.

In this example, calibration engine 30 includes memory 31, bus system 37, and processing device 35. Memory 31 stores calibrator 32 and clinical trial ML-based agent 33, which includes ML model 34. Clinical trial ML-based agent 33 also includes interface 33a (e.g., user interfaces/network interfaces/display or monitor interfaces, etc.) for clinical trial ML-based agent 33 to interface, communicate and so forth with natural language interface engine 36.

In this example, ML model 34 includes a pre-trained ML model and is used as the base model. Calibration engine 30 transmits the preprocessed data to calibrator 32. In turn, calibrator 32 uses the pre-processed data (e.g., question-answer pairs) to calibrate (e.g., fine-tune) ML model 34. Specific fine-tuning techniques like LoRA (Low-Rank Adaptation) are employed for efficiency. In LoRA, only a small subset of parameters is adjusted by decomposing large weight matrices into smaller, lower-rank matrices, significantly reducing the number of trainable parameters and allowing for efficient adaptation to new tasks while keeping the original model largely intact; essentially, it enables fine-tuning with minimal computation. To determine the lower rank matrices in LoRA, calibrator 32 identifies matrices where the "rank" (number of linearly independent rows or columns) is significantly lower than the maximum possible rank based on its size, essentially meaning the matrix contains redundancy and can be represented with fewer dimensions. In LoRA, these low-rank matrices are usually denoted as "A" and "B," which are multiplied together to approximate the desired weight updates instead of directly modifying the original model weights. A "matrix" refers to a collection of numbers arranged in rows and columns, but specifically, in the context of LoRA, it signifies a "low-rank matrix" - a smaller, more efficient representation of a larger weight matrix within a neural network, used to update the model's parameters while significantly reducing the number of trainable parameters during fine-tuning, allowing for faster and more memory-efficient training. Other fine-tuning techniques may also be employed, such as, e.g., parameter efficient fine-tuning, in-context fine-tuning, and so forth.

In some examples, the fine-tuning process is continuous. As new data is ingested and preprocessed, ML model 34 is updated. A schedule or trigger (e.g., new data available, every X hours) can be used to initiate the fine-tuning process. In other examples, to conserve memory and processing resources, the fine-tuning process is only initiated for changes that satisfy threshold criteria (e.g., are associated with a threshold weight), as previously described.

Natural language interface engine 36 interacts with trained (e.g., recently trained, trained based on data modifications, and so forth) ML model 34 through interface 33a of clinical trial ML-based agent 33. In some examples, natural language interface engine 36 includes a chatbot. Using client device 40, a user interacts with data processing system 16 through a chatbot interface, asking questions in natural language. Data for the chatbot interface is provided by natural language interface engine 36. That data is rendered on a display of client device 40. In this example, client device 40 transmits, via the interface, a query (e.g., a question) to natural language interface engine 36, which performs query processing. For example, the query is pre-processed (e.g., tokenization, stemming, and so forth). Natural language interface engine 36 includes tokenizer 38 that performs tokenization of the query.

For example, the query transmitted from client device 40 may include personally identifiable information (PII). In this example, tokenizer 38 replaces the PII with a non-sensitive substitute, known as a token. For example, tokenizer 38 is configured to replace the PII with a randomly generated string.

Tokenizer 38 then sends the pre-processed query to ML model 34 (e.g., which is finely tuned) and ML model 34 generates a response, which is transmitted to natural language interface engine 36. Natural language interface engine 36 presents a response of ML model 34 to the user in a clear and concise format.

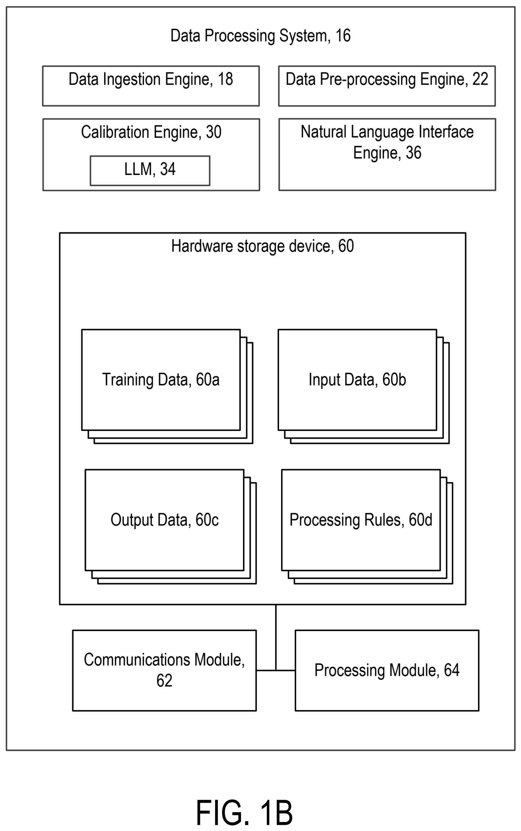

FIG. 1B shows various aspects of data processing system 16. In general, data processing system 16 includes several operation modules that perform particular functions related to the operation of data processing system 16. For example, data processing system 16 includes data ingestion engine 18, data pre-processing engine 22, calibration engine 30, and natural language interface engine 36. Further, data processing system 16 includes hardware storage device 60, communications module 62, and processing module 64. The operation modules can be provided as one or more computer executable software modules, hardware modules, or a combination thereof. For example, one or more of the operation modules can be implemented as blocks of software code with instructions that cause one or more processors of data processing system 16 to execute operations described herein. In addition, or alternatively, one or more of the operations modules can be implemented in electronic circuitry such as, e.g., programmable logic circuits, field programmable logic arrays (FPGA), or application specific integrated circuits (ASIC).

Hardware storage device 60 maintains information related to clinical trials. As an example, hardware storage device 60 can store training data 60a for training or prompting ML model 34 (FIG. 1A). In some implementations, training data 60a can include historical and/or ongoing audit trail data. In an example, audit trail data includes data specifying a site name, a participant ID and one or more actions that are performed with regard to that participant ID (e.g., one or more actions specifying that the participant's temperature has changed), and so forth.

As another example, hardware storage device 60 can store input data 60b that is used as an input to ML model 34 (FIG. 1A). As an example, input data 60b can include updates to information about participants in the clinical trial (e.g., updates to temperatures), queries, commands, instructions provided by a user, including information regarding a particular desired output of data processing system 16, and so forth. For instance, input data 60b can include information about a particular participant in a clinical trial, information regarding the participant of the clinical trial (e.g., the intervention that is being tested), the types of clinical trial that is to be performed (e.g., the "phase" of the clinical trial), the intended audience of the document (e.g., a particular government agency), and so forth.

Further, input data 60b can include information retrieved by data processing system 16 in support of fine-tuning ML model 34. As an example, input data 60b can include data retrieved from one or more drug information databases (e.g., information regarding a drug's composition, interactions between drugs, dosage of drugs, indications for use, side effects, etc.), e.g., when an update is made to those databases. Input data 60b can also include information retrieved from one or more data sources 14a...14n, e.g., when an update is made to data records (e.g., health records) in those data sources. In some examples, input data 60b includes updates made to specified fields in data record. For a given update, input data 60b includes information specifying the update and information specifying a key (e.g., a unique identifier representing a particular participant in the clinical trial) associated with the update (or a data record including the update) to enable ML model 34 to be calibrated not only for the update but also for the particular key for which the update occurred (e.g., thereby enabling a user to prompt ML model 34 for updated information about a particular clinical trial participant).

As another example, input data 60b can include data retrieved from one or more medical or scientific journals, e.g., when published or when an update is made. For instance, input data 60b can include one or more articles or other publications describing the use, safety, and/or efficacy of certain drugs or other medical interventions.

As another example, input data 60b can include data regarding one or more existing clinical trial protocols, e.g., when a modification or change is made. For instance, input data 60b can include data regarding series of steps, procedures, actions, or operations that were previously performed (e.g., by clinical trial researchers) to assess the safety and/or efficacy of particular medical interventions.

As another example, input data 60b can include data regarding one or more rules, regulations, and/or guidelines for performing clinical trials, e.g., when updates are made in one or more of data sources 14a...14n. For instance, input data 60b can include data regarding government rules, regulations and/or guidelines (e.g., as specified by a government agency, such as the Federal Drug Administration, FDA). Input data 60b can also include data regarding institutional rules, regulations and/or guidelines (e.g., as specified by a public or private hospital).

Further, hardware storage device 60 can store output data 60c generated by ML model 34. As an example, output data 60c can include one or more portions of content (e.g., text, images, charts, graphs, tables, etc.) generated by ML model 34 based on input data 60b. As another example, output data 60c can include responses generated by ML model 34 based on input data 60b.

Further, hardware storage device 60 can store processing rules 60d specifying how data in hardware storage device 60 can be processed to responses using ML model 34. As an example, processing rules 60d can include one or more rules for implementing, instruction tuning or prompting, and operating ML model 34 to produce output data 60c. For example, the one or more rules can specify that the training data 60a be provided to ML model 34 for training or prompting (e.g., such that ML model 34 can detect updates to clinical trial information, updates to participants' psychological or physical characteristics during the clinical trial, and so forth, and generate new output based on those detected updates).

As another example, the one or more rules can specify that input data 60b be provided to ML model 34 (e.g., to generate output data 60c representing a response to a query).

As another example, the one or more rules can specify that generated output data 60c be presented to the user and/or stored for future retrieval and/or processing (e.g., using the hardware storage device 60).

As another example, the one or more rules can specify one or more tools that facilitate the performance of particular actions by ML model 34. For example, the tools can specify certain actions or operations that can be performed by ML model 34 to retrieve data and to generate content based on the retrieved data. The one or more rules can also specify that when a change is detected in data sources 14a...14n to retrieve the changed data and one or more other types of data (that may be unchanged) to be included in the input data. Referring back to FIG. 1A, the rules may specify that when a change is detected in the "temp" field of table 51 to also retrieve corresponding data from the "time" field and the ID field for inclusion in the input data.

As described above, data processing system 16 also includes communications module 62. Communications module 62 allows for the transmission of data to and from data processing system 16. For example, communications module 62 can be communicatively connected to a network, such that it can transmit data to and receive data from a computer system, a system device, a data source and so forth. Information received from a computer system, a system device, a data source and so forth can be processed (e.g., using processing module 64) and stored (e.g., using hardware storage device 60).

As described above, data processing system 16 also includes processing module 64. Processing module 64 processes data stored or otherwise accessible to data processing system 16. For instance, processing module 64 can be used to execute one or more of the operations described herein (e.g., operations associated with ML model 34).

In some implementations, a software application can be used to facilitate performance of the tasks described herein. As an example, an application can be installed on client device 12 (FIG. 1A). Further, a user can interact with the application to input data and/or commands to data processing system 16, and review data generated by data processing system 16.

In one example, ML model 34 may be embodied as an artificial neural network. Artificial neural networks (ANNs) or connectionist systems are computing systems inspired by the biological neural networks that constitute animal brains. An ANN is based on a collection of connected units or nodes, called artificial neurons. Each connection, like the synapses in a biological brain, can transmit a signal from one artificial neuron to another. An artificial neuron that receives a signal can process it and then signal additional artificial neurons connected to it.

In common ANN implementations, the signal at a connection between artificial neurons is a real number, and the output of each artificial neuron is computed by some non-linear function of the sum of its inputs. The connections between artificial neurons are called 'edges'. Artificial neurons and edges may have a weight that adjusts as learning proceeds (for example, each input to an artificial neuron may be separately weighted). The weight increases or decreases the strength of the signal at a connection. Artificial neurons may have a threshold such that the signal is only sent if the aggregate signal crosses that threshold. The transfer functions along the edges usually have a sigmoid shape, but they may also take the form of other non-linear functions, piecewise linear functions, or step functions. Typically, artificial neurons are aggregated into layers. Different layers may perform different kinds of transformations on their inputs. Signals travel from the first layer (e.g., the input layer), to the last layer (e.g., the output layer), possibly after traversing the layers multiple times.

In general, a neural network is trained using a supervised learning technique based on training data, wherein the neural network is configured to receive training data as input (e.g., input data records) and to process the input to generate an output, e.g., that specifies whether PII is detected. In this example, the neural network includes a plurality of artificial neurons that are connected through edges and are aggregated into a plurality of neural network layers including at least an input layer and an output layer, wherein each of the edges is configured to transmit a signal from one artificial neuron to another artificial neuron, and wherein an output of each of the plurality of artificial neurons is computed based on inputs of the artificial neuron in accordance with a plurality of weights. In this example, new data is processed using the plurality of artificial neurons in the trained neural network in accordance with the values of the plurality of weights to detect PII, wherein the artificial neurons in the input layer are configured to receive the new data as input and the artificial neurons in the output layer are configured to generate a new output that specifies whether PII is detected.

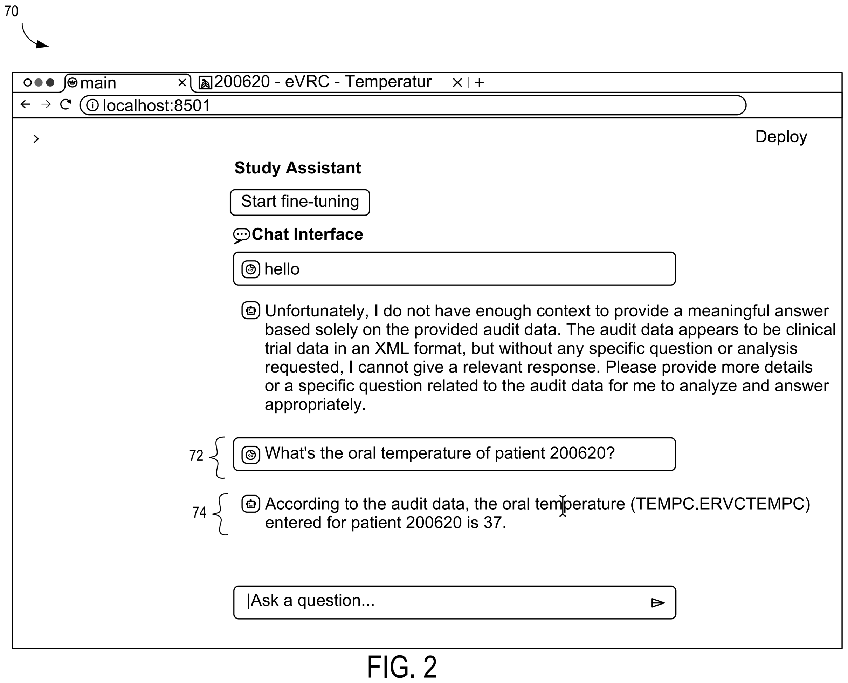

Referring to FIG. 2, graphical user interface 70 is shown for rendering a study assistant chat interface. Graphical user interface 70 may be rendered on client device 40 (FIG. 1A). The data for graphical user interface 70 may be transmitted from natural language interface engine 36 (FIG. 1A) to client deice 40. In this example, graphical user interface 70 includes portion 72 that displays input (e.g., text in the form of a query). In this example, the query specifies an identifier of "200620" and requests the temperature of the participant associated with that identifier. Data representing the query of portion 72 is transmitted to natural language interface 36, which in turn transmits via interface 33a the query to ML model 34. Using the techniques described herein, ML model 34 generates a response, which is rendered in portion 74 of graphical user interface 70.

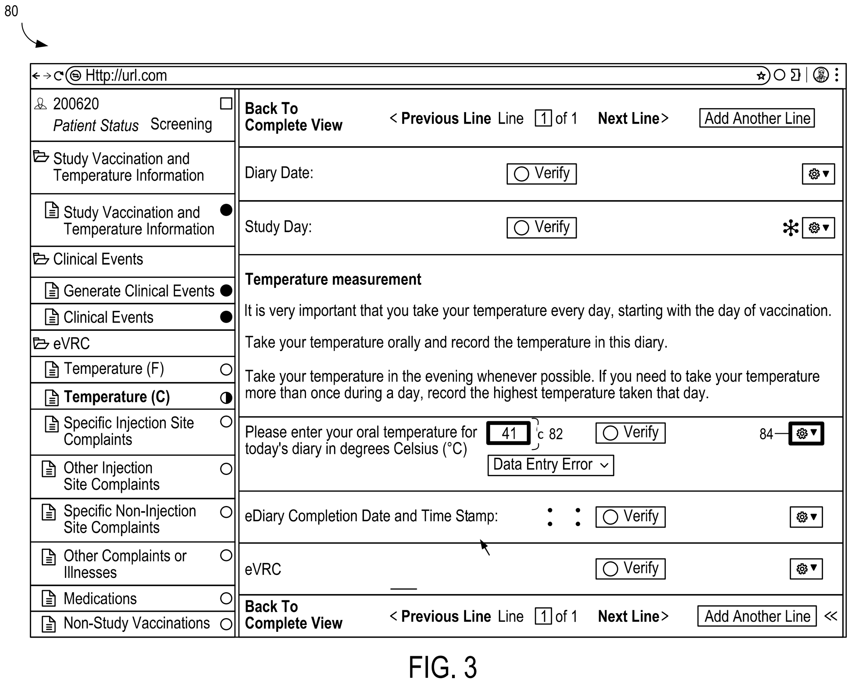

Referring to FIG. 3, graphical user interface 80 is shown. In this example, graphical user interface 80 is rendered by a client device, computer system, and so forth in communication with at least one of data sources 14a...14n. In this example, the temperature of the participant associated with the identifier of "200620" is updated to a value of forty-one degrees Celsius, as indicated in portion 82 of graphical user interface 80. Graphical user interface 80 also includes control 84, selection of which causes data representing the update entered into portion 82 to be stored in at least one of data sources 14a...14n. Using the techniques described herein, data ingestion engine 18 detects the update and determines that the update is of a type for which ML model 34 needs to be calibrated. As such, ML model 34 is calibrated in response to detecting the update, as described herein.

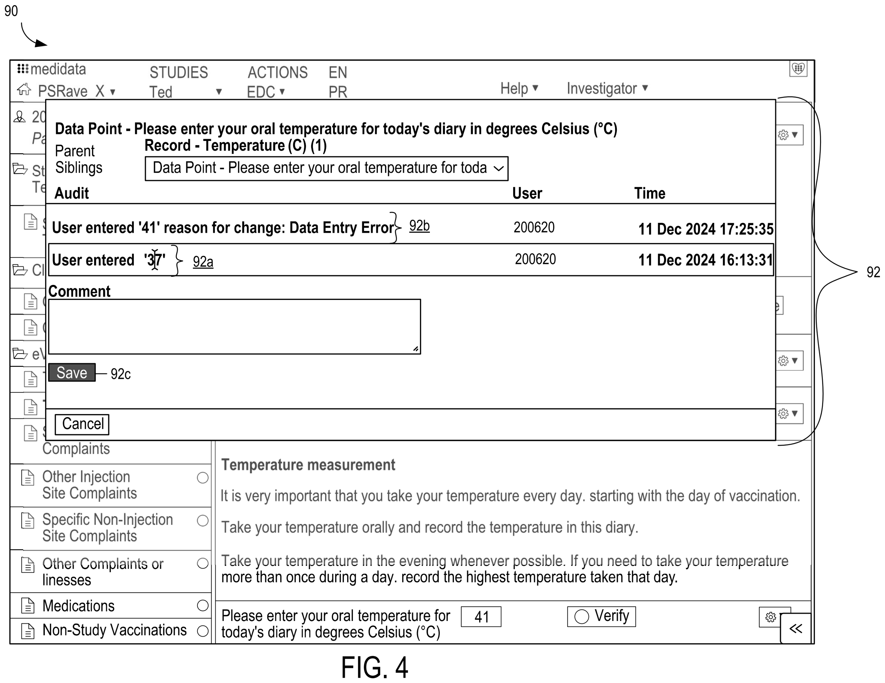

Referring to FIG. 4, graphical user interface 90 is shown of an audit trail that is generated, responsive to entry of the update illustrated in FIG. 3 into at least one of data sources 14a...14n. Graphical user interface 90 is rendered, e.g., on a display device of client device 12 in communication with at least one of data sources 14a...14n. In this example, data sources 14a...14n include a database management system (DBMS) that is configured to generate data records representing modifications to characteristics (e.g., temperature and other physical characteristics) to participants of a clinical trial, modifications to the clinical trial itself, and so forth.

In particular, graphical user interface 90 includes visualization 92 of the audit trail. Visualization 92 includes portion 92a representing a first (e.g., original) temperature entered for a participant associated with identifier 200620. Visualization 90 also includes portion 92b representing a second (e.g., updated) temperature entered for the participant associated with the identifier 200620. Visualization 90 also includes control 92c, selection of which causes the data represented in one or more of portions 92a, 92b to be committed to memory (e.g., to be stored in at least one of data sources 14a...14n (FIG. 1A), to be stored in at least one of tables 50, 51 (FIG. 1A) and so forth. In this example, upon selection of control 92c, table 51 (FIG. 1A) is updated with a row with cells specifying the identifier, the temperature and the time shown in portion 92b. Control 92c is rendered in juxtaposition to portions 92a, 92b of visualization 90.

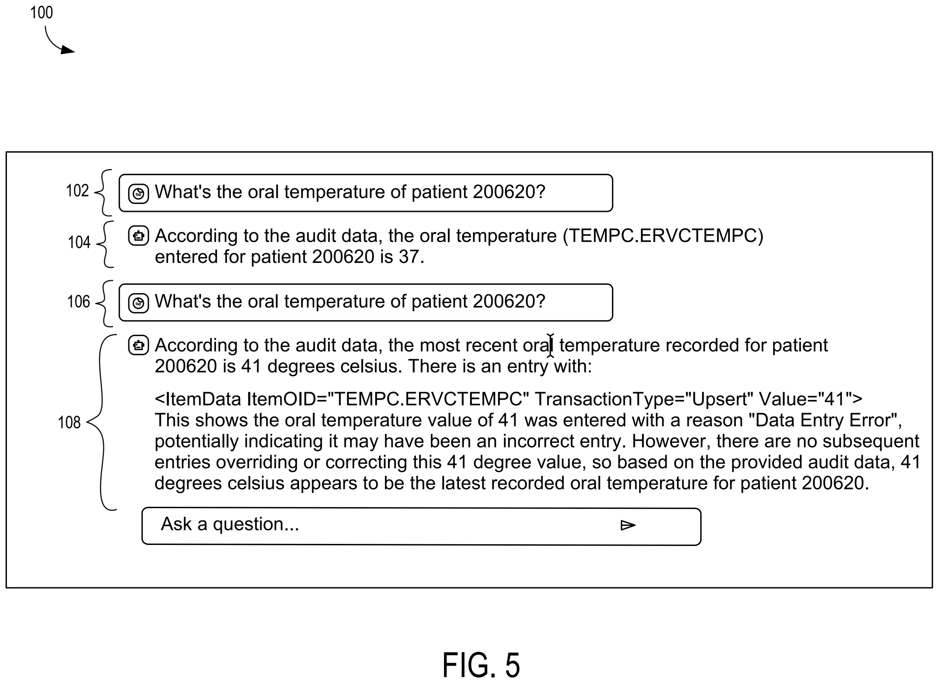

Referring to FIG. 5, graphical user interface 100 is shown. Graphical user interface 100 is rendered on a display device of client device 40 (FIG. 1A). In this example, graphical user interface 100 includes a chatbot through which a user of client device 40 inputs queries to data processing system 16. The chatbot also displays visualizations of output of data processing system 16.

Graphical user interface 100 includes portions 102, 104, 106, 108. Portion 102 includes an input portion, e.g., into which a user can input a query (i.e., what is the oral temperature of participant 200620?). Client device 40 transmits the query (e.g., data representing the input) to natural language interface engine 36, which - in turn- transmits the query, via interface 33a to ML model 34. ML model 34 returns a response, the contents of which are displayed in portion 104 of graphical user interface 100. Then, at a subsequent time, the user may enter into portion 106 another query. In this example, the query is entered into portion 106 at a time that is subsequent to a time in which a temperature for the participant associated with identifier 200620 is updated. ML model 34 having been trained on the update is able to provide a near real-time response as shown in portion 108. In this example, near real-time is with regard to when a user of client device 12 entered the updated temperature, as previously described. Generally, near real-time is with regard to when one or more modifications to data collected during a clinical trial are stored in a data store, input into a client device, input into a data processing system, and so forth.

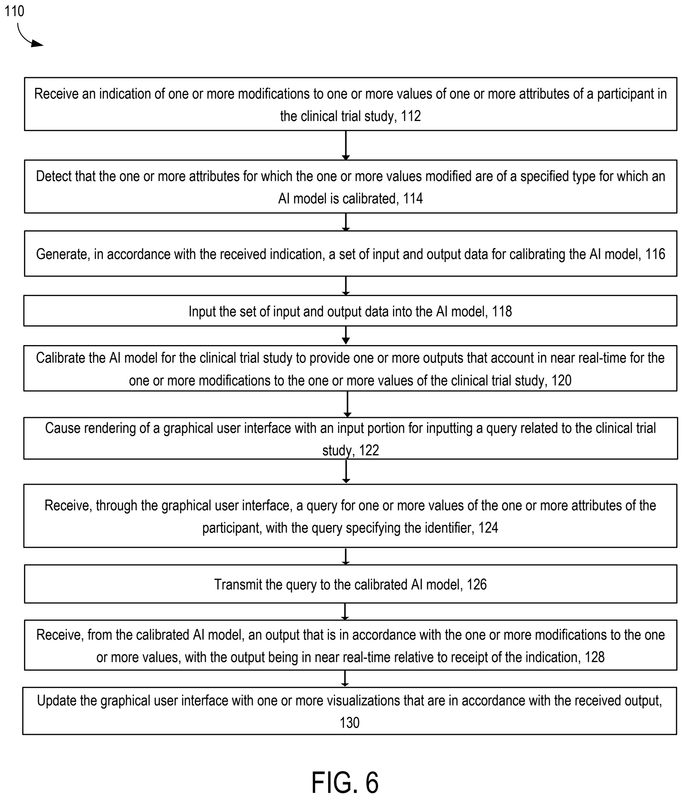

Referring to FIG. 6, example process 110 for calibrating a clinical trial ML-based agent is shown. The clinical trial ML-based agent is calibrated to provide near real-time outputs during a clinical trial study. In operation, a data processing system receives (112) an indication of one or more modifications to one or more values of one or more attributes of a participant in the clinical trial study, with the indication specifying an identifier representing the participant in the clinical trial study. For example, the data processing system receives an indication that a temperature of a participant associated with a given identifier has changed.

The data processing system detects (114) that the one or more attributes for with the one or more modified values are of a specified type for which an artificial intelligence (AI) model is calibrated. Responsive to the detecting, the data processing system generates (116), in accordance with the received indication, a set of input and output data for calibrating the AI model, with the output specifying an appropriate response to the input, and with the input being in accordance with the one or more attributes for which the one or more values are modified. The data processing system inputs (118) the set of input and output data into the AI model. Based on the inputting of the set of input and output data, the data processing system calibrates (120) the AI model for the clinical trial study to provide one or more outputs that account in near real-time for the one or more modifications to the one or more values of the clinical trial study. For example, the data processing system may calibrate the AI model by calibrating clinical trial ML-based agent 33 (FIG. 1A) that includes ML model 34 (FIG. 1A).

The data processing system causes (122) rendering of a graphical user interface with an input portion for inputting a query related to the clinical trial study. The data processing system receives (124), through the graphical user interface, a query for one or more values of the one or more attributes of the participant, with the query specifying the identifier. The data processing system transmits (126) the query to the calibrated AI model. The data processing system receives (128), from the calibrated AI model, an output that is in accordance with the one or more modifications to the one or more values, with the output being in near real-time relative to receipt of the indication. The data processing system updates (130) and/or causes to be updated the graphical user interface with one or more visualizations that are in accordance with the received output, wherein the one or more visualizations are juxtaposed to the input portion.

In some examples, the one or more attributes are one or more first attributes and the identifier is a primary key. The data processing system generates the set of input data, e.g., by identifying one or more second attributes to be included in the set of input data and searching data structures (e.g., tables 50, 51 in FIG. 1A) stored in memory for one or more foreign keys with a foreign-primary key relationship to the primary key, e.g., as specified by cells 50a, 51a (FIG. 1A). Based on the searching, the data processing system identifies one or more data structures (e.g., tables 50, 51) stored in memory with one or more foreign keys (e.g., as specified by cell 50a) with a foreign-primary key relationship (as specified by the cardinality indicator in FIG. 1A) to the primary key (e.g., as specified by cell 51a). The data processing system then retrieves, from those one or more identified data structures, one or more values of the one or more second attributes, with the one or more retrieved values being associated with the foreign key and generates input data in accordance with one or more modified values of the one or more first attributes and one or more values of the one or more second attributes.

### Example Computer Systems

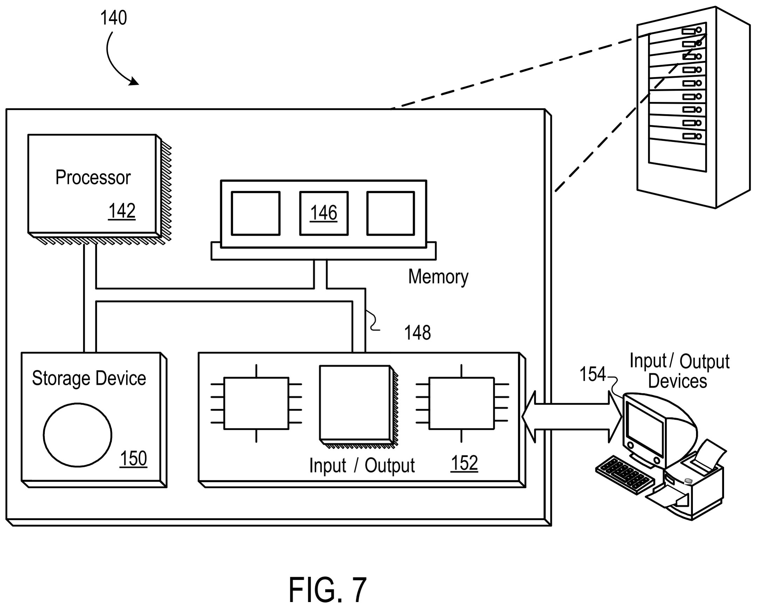

FIG. 7 depicts an example computing system, according to implementations of the present disclosure. The system 140 may be used for any of the operations described with respect to the various implementations discussed herein. The system 140 may include one or more processors 142, a memory 146, one or more storage devices 150, and one or more input/output (I/O) devices 154 controllable through one or more I/O interfaces 152. The various components 142, 146, 150, 152, or 154 may be interconnected through at least one system bus 148, which may enable the transfer of data between the various modules and components of the system 140.

The processor(s) 142 may be configured to process instructions for execution within the system 140. The processor(s) 142 may include single-threaded processor(s), multi-threaded processor(s), or both. The processor(s) 142 may be configured to process instructions stored in the memory 146 or on the storage device(s) 150. The processor(s) 142 may include hardware-based processor(s) each including one or more cores. The processor(s) 142 may include general purpose processor(s), special purpose processor(s), or both.

The memory 146 may store information within the system 140. In some implementations, the memory 146 includes one or more computer-readable media. The memory 146 may include any number of volatile memory units, any number of non-volatile memory units, or both volatile and non-volatile memory units. The memory 146 may include read-only memory, random access memory, or both. In some examples, the memory 146 may be employed as active or physical memory by one or more executing software modules.

The storage device(s) 150 may be configured to provide (e.g., persistent) mass storage for the system 140. In some implementations, the storage device(s) 150 may include one or more computer-readable media. For example, the storage device(s) 150 may include a floppy disk device, a hard disk device, an optical disk device, or a tape device. The storage device(s) 150 may include read-only memory, random access memory, or both. The storage device(s) 150 may include one or more of an internal hard drive, an external hard drive, or a removable drive.

One or both of the memory 146 or the storage device(s) 150 may include one or more computer-readable storage media (CRSM). The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 140. In some implementations, the CRSM may include a data store that provides storage of computer-readable instructions or other information in a non-transitory format. The CRSM may be incorporated into the system 140 or may be external with respect to the system 140. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 142 and the memory 146 may be supplemented by, or incorporated into, one or more application-specific integrated circuits (ASICs).

The system 140 may include one or more I/O devices 154. The I/O device(s) 154 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 154 may also include one or more output devices such as a display, LED(s), an audio output device (e.g., a speaker), a printer, a haptic output device, and so forth. The I/O device(s) 154 may be physically incorporated in one or more computing devices of the system 140 or may be external with respect to one or more computing devices of the system 140.

The system 140 may include one or more I/O interfaces 152 to enable components or modules of the system 140 to control, interface with, or otherwise communicate with the I/O device(s) 154. The I/O interface(s) 152 may enable information to be transferred in or out of the system 140, or between components of the system 140, through serial communication, parallel communication, or other types of communication. For example, the I/O interface(s) 152 may comply with a version of the RS-232 standard for serial ports, or with a version of the IEEE 1284 standard for parallel ports. As another example, the I/O interface(s) 152 may be configured to provide a connection over Universal Serial Bus (USB) or Ethernet. In some examples, the I/O interface(s) 152 may be configured to provide a serial connection that is compliant with a version of the IEEE 1394 standard.

The I/O interface(s) 152 may also include one or more network interfaces that enable communications between computing devices in the system 140, or between the system 140 and other network-connected computing systems. The network interface(s) may include one or more network interface controllers (NICs), or other types of transceiver devices configured to send and receive communications over one or more networks using any network protocol.

Computing devices of the system 140 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 140 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on its software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random-access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

### EMBODIMENTS

Although the present invention is defined in the claims, it should be understood that the present invention can also (alternatively) be defined in accordance with the following embodiments:
1. A method implemented by a data processing system for calibrating an artificial intelligence model to provide near real-time outputs during a clinical trial study, comprising:
   receiving, at a data processing system, an indication of one or more modifications to one or more values of one or more attributes of a participant in the clinical trial study, with the indication specifying an identifier representing the participant in the clinical trial study;
   detecting that the one or more attributes for which the one or more values are modified are of a specified type for which an artificial intelligence (AI) model is calibrated;
   responsive to the detecting, generating, in accordance with the received indication, a set of input and output data for calibrating the AI model, with the output specifying an appropriate response to the input, and with the input being in accordance with the one or more attributes for which the one or more values are modified;
   inputting the set of input and output data into the AI model;
   based on the inputting of the set of input and output data, calibrating the AI model for the clinical trial study to provide one or more outputs that account in near real-time for the one or more modifications to the one or more values of the clinical trial study;
   causing rendering of a graphical user interface with an input portion for inputting a query related to the clinical trial study;
   receiving, through the graphical user interface, a query for one or more values of the one or more attributes of the participant, with the query specifying the identifier;
   transmitting the query to the calibrated AI model;
   receiving, from the calibrated AI model, an output that is in accordance with the one or more modifications to the one or more values, with the output being in near real-time relative to receipt of the indication; and
   updating the graphical user interface with one or more visualizations that are in accordance with the received output, wherein the one or more visualizations are juxtaposed to the input portion.
2. The method of embodiment 1, wherein the one or more attributes are one or more first attributes, wherein the identifier is a primary key, and wherein generating the set of input data comprises:
   identifying one or more second attributes to be included in the set of input data;
   searching data structures stored in memory for one or more foreign keys with a foreign-primary key relationship to the primary key;
   based on the searching, identifying one or more data structures stored in memory with one or more foreign keys with a foreign-primary key relationship to the primary key;
   retrieving, from those one or more identified data structures, one or more values of the one or more second attributes, with the one or more retrieved values being associated with the foreign key;
   generating input data in accordance with one or more modified values of the one or more first attributes and one or more values of the one or more second attributes.
3. The method of embodiment 1, further comprising: continuously calibrating the AI model during the clinical trial study, based on detection of one or more additional modifications of one or more specified types.
4. The method of embodiment 1, wherein the calibrating includes fine-tuning the AI model with the set of input and output data.
5. The method of embodiment 1, further comprising:
   storing optimization rules specifying one or more types of modifications or one or more types of attributes that trigger a calibration of the AI model; and
   executing the optimization rules.
6. The method of embodiment 1, further comprising:
   polling a hardware storage device for new audit data, with new audit data being audit data stored in the hardware storage device after a specified time.
7. The method of embodiment 1, wherein the calibrating comprises:
   training one or more specified parameters of the AI model in accordance with the generated set; or
   performing in-context fine-tuning of the AI model in accordance with the generated set.
8. A system for calibrating an artificial intelligence model to provide near real-time outputs during a clinical trial study, comprising:
   at least one processor; and
   a memory communicatively coupled to the at least one processor, the memory storing instructions which, when executed by the at least one processor, cause the at least one processor to perform the method of any of embodiments 1-7.
9. One or more non-transitory computer-readable media for calibrating an artificial intelligence model to provide near real-time outputs during a clinical trial study, the one or more non-transitory computer-readable media storing instructions which, when executed by at least one processor, cause the at least one processor to perform the method of any of embodiments 1-7.

## Claims

1. A method (110) implemented by a data processing system for calibrating an artificial intelligence model to provide near real-time outputs during a clinical trial study, comprising:
receiving (112), at a data processing system, an indication of one or more modifications to one or more values of one or more attributes of a participant in the clinical trial study, with the indication specifying an identifier representing the participant in the clinical trial study;
detecting (114) that the one or more attributes for which the one or more values are modified are of a specified type for which an artificial intelligence (AI) model is calibrated;
responsive to the detecting, generating (116), in accordance with the received indication, a set of input and output data for calibrating the AI model, with the output specifying an appropriate response to the input, and with the input being in accordance with the one or more attributes for which the one or more values are modified;
inputting (118) the set of input and output data into the AI model;
based on the inputting of the set of input and output data, calibrating (120) the AI model for the clinical trial study to provide one or more outputs that account in near real-time for the one or more modifications to the one or more values of the clinical trial study;
causing (122) rendering of a graphical user interface with an input portion for inputting a query related to the clinical trial study;
receiving (124), through the graphical user interface, a query for one or more values of the one or more attributes of the participant, with the query specifying the identifier;
transmitting (126) the query to the calibrated AI model;
receiving (128), from the calibrated AI model, an output that is in accordance with the one or more modifications to the one or more values, with the output being in near real-time relative to receipt of the indication; and
updating (130) the graphical user interface with one or more visualizations that are in accordance with the received output, wherein the one or more visualizations are juxtaposed to the input portion.

2. The method (110) of claim 1, wherein the one or more attributes are one or more first attributes, wherein the identifier is a primary key, and wherein generating the set of input data comprises:
identifying one or more second attributes to be included in the set of input data;
searching data structures stored in memory for one or more foreign keys with a foreign-primary key relationship to the primary key;
based on the searching, identifying one or more data structures stored in memory with one or more foreign keys with a foreign-primary key relationship to the primary key;
retrieving, from those one or more identified data structures, one or more values of the one or more second attributes, with the one or more retrieved values being associated with the foreign key;
generating input data in accordance with one or more modified values of the one or more first attributes and one or more values of the one or more second attributes.

3. The method (110) of any one of claims 1 or 2, further comprising continuously calibrating the AI model during the clinical trial study based on detection of one or more additional modifications of one or more specified types.

4. The method (110) of any one of claims 1 to 3, wherein calibrating (120) the AI model for the clinical trial study includes fine-tuning the AI model with the set of input and output data.

5. The method (110) of any one of claims 1 to 4, further comprising:
storing optimization rules specifying one or more types of modifications or one or more types of attributes that trigger a calibration of the AI model; and
executing the optimization rules.

6. The method (110) of any one of claims 1 to 5, further comprising:
polling a hardware storage device for new audit data, with new audit data being audit data stored in the hardware storage device after a specified time.

7. The method (110) of any one of claims 1 to 6, wherein calibrating (120) the AI model for the clinical trial study comprises:
training one or more specified parameters of the AI model in accordance with the generated set; or
performing in-context fine-tuning of the AI model in accordance with the generated set.

8. A system (140) for calibrating an artificial intelligence model to provide near real-time outputs during a clinical trial study, comprising:
at least one processor (142); and
a memory (146) communicatively coupled to the at least one processor, the memory storing instructions which, when executed by the at least one processor, cause the at least one processor to perform the method (110) of any of claims 1 to 7.

9. One or more non-transitory computer-readable media for calibrating an artificial intelligence model to provide near real-time outputs during a clinical trial study, the one or more non-transitory computer-readable media storing instructions which, when executed by at least one processor (142), cause the at least one processor to perform the method (110) of any of claims 1 to 7.
